# EUROPEAN PATENT SPECIFICATION

(11) **EP 2 155 299 B1**
(45) Date of publication and mention of the grant of the patent: **26.01.2022**
(21) Application number: 07847877.3
(22) Date of filing: 06.12.2007
(51) Int. Cl.: A61M 5/142, A61M 25/02, A61M 5/14, A61M 5/158

(54) **DELIVERY DEVICE**
ABGABEVORRICHTUNG
DISPOSITIF DE DISTRIBUTION

(30) Priority: 07.05.2007 US 928025 P
(43) Date of publication of application: 24.02.2010
(73) Proprietor: Unomedical A/S, 3460 Birkerød (DK)
(72) Inventor: GYRN, Steffen, DK-4100 Ringsted (DK); MATHIASEN, Orla, DK-4180 Sorø (DK); PEDERSEN, Tenna, M., DK-2000 Frederiksberg (DK)
(74) Representative: Wilson Gunn
(86) International application number: PCT/EP2007/063389
(87) International publication number: WO 2008/135098

(56) References cited:
- WO-A-2006/015600
- WO-A-2006/032692
- WO-A-2008/014791
- US-A1- 2004 158 207

## Description

The present invention relates to a delivery device for delivering medication to a patient under controlled conditions, and a system with an inserter and delivery device.

### Prior art

Often delivery devices for intermittent or continuous administration of a therapeutical substance, such as insulin, are in form of a two-part device. Such a traditional delivery device comprises a base part having a cannula for subcutaneous insertion into a patient and comprising means for fastening of the base part to the patients skin, further the base part has means for closing of fluid access to the base part and it has means for opening of fluid access e.g. for receiving a connector cannula extending from a connector part and for bringing the connector cannula into fluid communication with the cannula of the base part. Often, the connector part is in fluid communication with a drug delivery device, e.g. an insulin pump.

Different kinds of delivery devices are described in WO 02/068014 A2, EP 0 956 879 A1, US 5 522 803, US 2003/0225373 A1 and WO 03/026728 A1.

US 2003/0176852A1 discloses a delivery device in which a base part comprises a pivoting member, said base part comprising a cannula for insertion into a patient and pivoting member has an inner cavity with one receiving end adapted to receive an inserter needle or a connector cannula and two connecting ends (3161 and 320) for further connection with the cannula of the base part. During insertion the pivoting member is positioned orthogonal to the base part and an inserter needle penetrates a membrane in the receiving end and the needle passes through a canal and through the first connecting end into the cannula which then can be inserted. After insertion the needle is removed and the pivoting member is connected with a connector. The connector and the pivoting member are connected from the same direction as the connection between the pivoting member and the inserter. The pivoting member is then turned in order for the second connecting end to align with the cannula. This device has the drawback that it is very sensitive to movement of the pivoting member since a small movement will close of the delivery of drugs.

WO 02/094352 A2 discloses a delivery device having in the base part a construction that makes it possible to receive an insertion needle from one direction and a connector needle from a second direction. This design does not allow the patient to choose from which direction he/she wants to connect the connector with the base part.

In these prior art delivery devices the construction of the cannula and the means for providing fluid communication between the cannula and the cannula from the connector is unique for each set. Normally each infusion set also utilizes a specific set of guiding and/or locking means thus allowing only for a specific connector to engage with the base part.

WO 06/015600 A1 discloses a delivery device having a universal part having a cannula and means adapted to receive the cannula from the connector and fitting to most/all common infusion sets were available. This design allows for different types of connectors to be used with the same base part and visa versa, and it will also be possible to connect the connector from different angles.

US 2004/158207 discloses a device for inserting a cannula into tissue, including a cannula, a protective element which can accommodate said cannula, an operating element for moving the cannula out of the protective element, and a holder fixedly connected to the cannula. The invention encompasses a system for connecting a liquid supply to the cannula.

WO 2006/032692 A1 discloses a medical device is provided comprising a cannula (771, 861) having a distal end portion adapted to be arranged through the skin of the subject and having a distal opening, and a needle (761, 861) arranged coaxially with and being axially moveable relative to the cannula, the needle comprising a pointed distal end, wherein the device is adapted for advancing the cannula with the distal end of the needle projecting there from through the dermis of the subject, and further advancing the cannula into the subcutis with the distal end of the cannula projecting relative to the needle.

According to the present invention, there is provided a delivery device according to claim 1.

The cannula device for mounting in a base part may comprise a housing and at least one membrane together defining at least one cavity, the cannula device further comprising a cannula mounted in the housing and being in fluid communication with the at least one cavity, which cannula device is provided with means for attaching the device to the base part on the proximal side of the device.

The advantage of such a cannula device is that it can be used as a standard component in delivery devices whether the delivery device has inclined or orthogonal insertion of the cannula. Thus this standard component can be mass produced and be used as a component in series of desired designs of the delivery devices. This results in lower manufacturing costs, a more flexible production line and a more flexible product. The positioning of the attaching means on the proximal side, i.e. the side turned towards the patient after mounting, of the device makes it easier to position the cannula device correctly by insertion as it is possible to cover or connect the sides of the cannula device with a handle or inserter device. Thus the attaching means will assure the attachment to the base part or receiving part while the side parts of the attaching means will assure the adaptation to the insertion device.

In another embodiment the cannula device for mounting in a base part comprises a housing and at least one membrane together defining at least one cavity, the cannula device further comprises a cannula mounted in the housing and being in fluid communication with the at least one cavity, where the cannula device is provided with means for attaching the cannula device unreleasably to the base part, i.e. to a specially adapted receiving portion of the base part.

The cannula device is normally a disposable device which is thrown away after use as the cannula is in contact with the patient's blood. If the base part to which the cannula device is attached also is a disposable device with approximately the same operating life it will not be necessary to be able to remove the device from the base part as both cannula device and base part will be removed and disposed of normally after having been used for a few days. When it is not possible to remove the cannula device from the base part it is not possible to have a used cannula device confused with a new sterile cannula device and it will also be evident that the receiving portion of the base part in which a cannula device is locked is not suitable for use.

If the base part is of a type which can be attached to the patient for a longer period, it might be possible to insert a new cannula device at a different position while the used cannula device is removed from the subcutaneous position e.g. by removing the cannula device together with a receiving portion or a part of a receiving portion to which it might be permanently attached.

Both have the advantage that it is possible for the user first to carefully position the base part, and after having positioned the base part properly, then the user can concentrate on injecting the cannula device.

In one embodiment the means for attaching the device to the base part comprise mechanical features cooperating with corresponding means on the base part, e.g. the means for attaching the device to the base part comprise parts extending from a proximal surface of the cannula device which parts can pivot and thereby temporarily reduce the diameter in at least one position or the means for attaching the device to the base part can comprise an adhesive surface on a proximal surface of the cannula device adhering to a corresponding surface of the base part.

In one embodiment the cannula device is inserted with an inserter device provided with a covering part covering the full length of the cannula device.

In one embodiment the part of the body of the cannula device having the largest diameter is rotational-symmetrical around a central axis.

In another embodiment the part of the body of the cannula device having the largest diameter has angled sides e.g. providing a triangular or quadrangular profile when cut-through. When having angled sides the profile of the cannula device can be used to define the correct insertion position.

In one embodiment the cannula device comprise a body showing a smooth outer surface and having an inner cavity, the inner cavity is at the distal end covered with a wall such as a membrane or a septum which can be penetrated by a needle such as a connector needle or a syringe and at the proximal end of the inner cavity a cannula is embedded, the outer proximal surface of the body, i.e. a surface of the body facing the receiving portion during injection of the cannula device, is provided with means for unreleasably attaching the device to a receiving portion. The smooth outer surface can e.g. have a round or oval circumference and the wall covering the distal end of the inner cavity can be penetrated either by a pointy or by a blunt needle which ever might be preferred.

In one embodiment the unreleasable attachment between the receiving portion and the cannula device is formed automatically, that is without the need to take any action in order to form the unreleasable attachment, as the cannula device is pushed against the receiving portion.

The delivery device includes a base part provided with a receiving portion for a cannula device where the receiving section may have guiding means for an inserter device which inserter device holds the cannula device before insertion, i.e. the receiving portion has no guiding means for the cannula device or at least the receiving portion does not need guiding means for the cannula device as the guiding means for the inserter device might provide sufficient guidance for correct positioning of the cannula device. In one embodiment the cannula device corresponds to an internal opening in a part of the inserter and the cannula device is provided with means for attaching the device to the base part on the proximal side of the body of the cannula device.

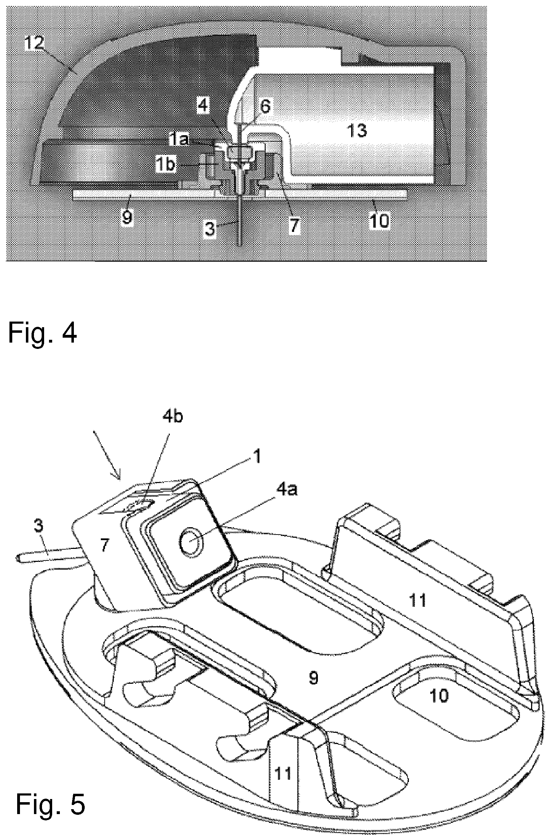

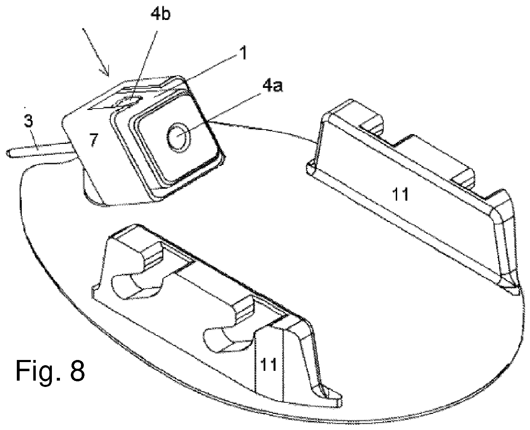

The delivery part which can be connected to this base part may have more than one position relative to the attachment parts. In a first position parts of the delivery part corresponding to the attachment parts of the base part is/are brought into contact with the attachments parts, after contact is made the parts of the delivery part corresponding to the attachment parts of the base part slides along a track or a surface towards a second position where a fluid connection between the delivery part and the cannula device) is formed. The tracks or surface can be a continuous opening in an upright wall as shown e.g. in fig. 5 or 8, or it can be the outer/upper of a protruding surface leading towards the receiving portion. If the tracks or surface is a continuous opening in an upright wall, then the corresponding parts of the delivery part could be protruding parts engaging the openings. If the tracks or surface is a band then the corresponding parts of the delivery part are corresponding sliding parts.

The device will be described in further detail with reference to the figures:
FIG. 1 A is a view of a cannula device, B is a cut-through view of the same cannula device as shown in A, C is a cut-through view along line A-A of fig. 2 of a cannula device having a square profile, D shows a cannula device having a square profile and two access positions from different angles;
FIG. 2 is a cut-through view of a cannula device placed in a receiving portion of a delivery device;
FIG. 3 is a view from above of a base part on which a top comprising a reservoir and means for transporting the content of the reservoir to the patient has been mounted;
FIG. 4 is a cut-through view of a delivery device shown in fig. 3;
FIG. 5 shows a base part having a close fit square receiving section for a square cannula device with two access openings;
FIG. 6 shows a cannula device with two access openings placed in an inserter positioned in contact with a base part:
FIG. 7 shows a base part having a centrally positioned receiving portion adapted to a cannula device having to access openings.
FIG. 8 shows a base part of a similar type as the one shown in fig. 7 but in fig. 8 the base part has the form and size of a credit card without a separate mounting pad.

### DETAILED DESCRIPTION OF THE INVENTION

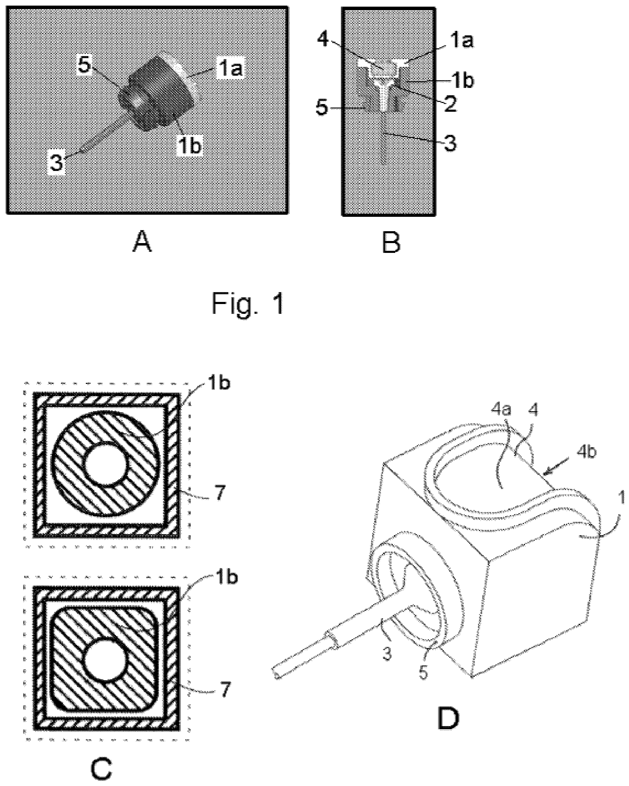

FIG. 1 A and B show an embodiment not covered by the claimed invention , the cannula device includes a housing 1a, 1b and a wall in the form of e.g. a membrane 4 which together define an inner cavity adapted to receive a piercing member 6 extending from e.g. a connector or a syringe. The housing 1a, 1b is normally made of a relatively hard molded plastic material.

The lower part 1b can be constructed of a cylindrical upper part where the inner surface forms the walls of the inner cavity and the outer surface is smooth and without protrusions, and of a cylindrical lower part with a smaller diameter where the inner surface forms an opening which supports a cannula 3 and the outer surface comprise means 5 for attaching the cannula device unreleasably to a portion 9 of the base part which portion 9 can be placed on the skin of a patient.

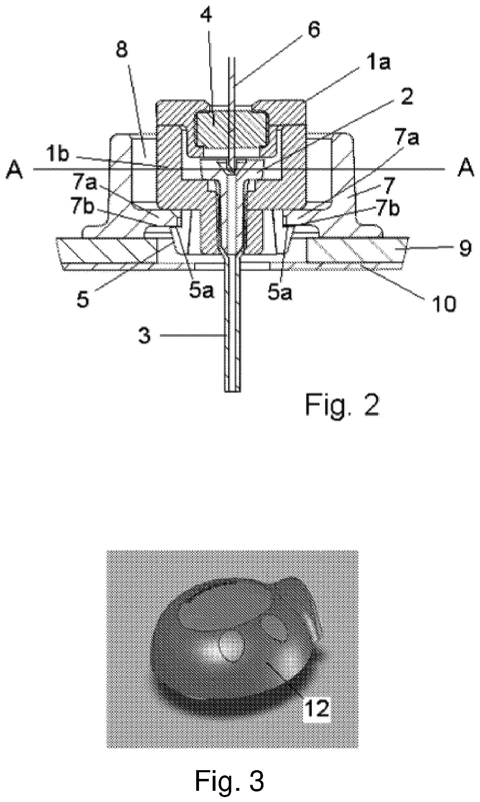

The cannula device can also be constructed with an angular profile e.g. a quadrangular profile as shown in fig. 1 C. This figure shows two cannula devices: a device having a round profile (upper) and a device having a square profile (lower). This profile shows when the cannula device is seen from above along the line A-A shown in fig. 2. Whether the profile of the cannula device is round or angular it might have a loose fit or a close fit in the receiving portion where a loose fit and transporting means for transferring fluid from the reservoir normally indicates that the receiving section is provided with guiding means for the inserter and the cannula device is placed in correct position when the inserter is placed according to the guiding means and when the inserter is removed from the receiving section an empty space corresponding to the walls of the inserter can appear around the cannula device. A close fit means that the receiving section of the base part is provided with a room closely corresponding to the form or the profile of the cannula device and the cannula device is positioned inside the room, e.g. by an inserter, which room has walls closely corresponding to the outer walls of the cannula device.

The cannula device shown in fig. 1 D (covered by the claimed invention) has two access openings covered with one membrane 4, in another (not shown) embodiment each access opening could be covered with separate non-connected membranes at different surface positions (e.g. respectively 4a and 4b). Such a cannula device can be fed with medication from two different angles via the surface 4a or the surface 4b. Such a device provides the possibility of having an extra access for medication if a corresponding opening is provided in the receiving portion 7 or alternatively the cannula device could be a standard device for two different types of base parts provided with different openings in the receiving portion 7.

The cannula is made of a soft inert material and in this embodiment the cannula 3 is attached to the housing 1b by pushing a fastening part 2 made of a more rigid material than the cannula 3 into the opening of the cylindrical lower part after positioning the cannula 3 in the opening. As the fastening part 2 is pushed into the opening the cannula 3 will be squeezed against the walls of the opening and this pressure will keep the cannula 3 in a correct position.

The means 5 for attaching the cannula device unreleasably to the base part is in this embodiment constructed as several hooks 5; these hooks 5 can pivot around the position where they are attached to the housing la, in this embodiment the attachments for the hooks 5 are of a flexible material i.e. the hooks can be pushed inwards when the hooks 5 pass an area of reduced diameter. Each hook 5 is provided with an upper surface 5a parallel to a contact surface 7b of a receiving portion 7 of the base part 9. Each hook 5 is also provided with an inclined surface 5b which inclined surface during insertion of the cannula device is in contact with a protruding part 7a of the receiving portion 7. When the cannula device is pushed down into the receiving portion 7 the hooks 5 are pushed inward against the lower cylindrical part of the housing 1b and as the hooks 5 in this position are biased, the hooks 5 will return to their original position when the inclined surface 5b of the hooks 5 has fully passed the protruding part 7a of the receiving portion 7. When the hooks 5 return to their original position the upper surface 5a of the hooks will be in touch with the contact surfaces 7b of the receiving portion 7 and the cannula device will be locked in this position as neither the cannula device nor the receiving portion 7 are provided with means to push the hooks 5 inward against the lower cylindrical part of the housing 1b.

According to another not shown embodiment either the upper side of the protruding parts 7a or the lower side of the housing 1a is provided with an adhesive which adhesive then works as unreleasably attaching means when the cannula device is pushed into position in the receiving portion 7.

Fig. 2 shows the cannula device of fig. 1, where the cannula device is positioned in the receiving portion 7. The receiving portion 7 is provided with essentially vertically positioned walls covering the side section of the cannula device and a bottom part formed by the protruding parts 7a on which the cannula device rests when locked in the receiving portion 7. The essentially vertical walls which encircle or surround the cylindrical space 8 around the cannula device can create a guiding mean for an inserter. The cannula device is in this embodiment fully covered by a lower cylindrical part of the inserter and when the user wants to inject the cannula device, the cylindrical lower part of the inserter is placed in the space 8 formed by the receiving portion 7 and then the cannula device is pushed in position by a plunger being moved forward inside the cylindrical lower part of the inserter.

The receiving portion 7 is attached unreleasably to the portion 9 of the base part which portion 9 is fastened to the skin of a patient e.g. with a mounting pad 10.

Fig. 3 shows an upper view of a base part wherein the base part 9 has been provided with a cover 12 in which a delivery part comprising a reservoir 13 for medication and means for transporting of the medication from the reservoir to the patient are embedded.

Fig. 4 shows a cut-through view of the device shown in fig. 3 not covered by the present invention. Fig. 4 shows a receiving portion 7 positioned on a base part which base part has a portion 9 provided with an underlying mounting pad 10. A cannula device has been inserted in the receiving portion 7 and the cannula 3 of the cannula device is inserted subcutaneously in a patient. The cannula device and the receiving portion 7 could be either square or round. The cover 12 is mounted releasably on the base part 9, and a connector needle 6 forms a fluid connection between the reservoir 13 which is attached to the inside of the cover 12 and the cannula device by penetrating the septum 4 of the cannula device. The transporting means are not shown.

The delivery device of fig. 4 can be mounted on the patient through the following steps:
I. A sterile base part is unpacked and secured to the skin of the patient.
II. A sterile single-use inserter including a cannula device is unpacked or a sterile part comprising an injection needle combined with a cannula device is unpacked and applied to a multiple-use inserter, the proximal end of the inserter is placed in the guiding means 8 of the receiving portion 7 and the cannula device is inserted, i.e. the cannula 3 is injected subcutaneously.
III. A delivery part comprising a cover 12, a reservoir 13 and means for transporting the content of the reservoir to the patient is fastened to the base part, and when the cover 12 is fastened to the base part the connector needle 6 penetrates the septum 4 of the cannula device and then the delivery device is ready to work.

Fig. 5 shows an embodiment of a delivery device according to the present invention wherein the receiver portion 7 makes it possible to inject liquid from to different angles. The cannula device has as shown in fig. 1 D two access openings but in the cannula device of fig. 5 each opening is covered by a separate membrane 4b and 4a. The opening covered by the first membrane 4a can e.g. be used when applying the cover 12 inside which a fluid connection is formed to a reservoir 13 while the second access covered by the second membrane 4b e.g. can be used when another medication or a higher dose than the one flowing through the fluid connection from the reservoir 13, has to be injected. In this situation the cover 12 could be provided with a corresponding opening which would make it possible to inject extra or other kinds of medication without removing the cover.

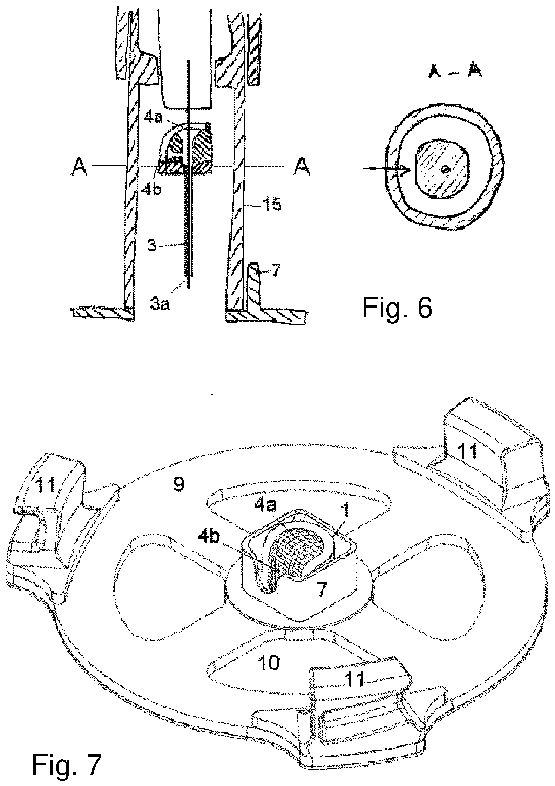

Fig. 6 shows an embodiment of a cannula device intended for a base part having a centrally positioned receiving portion 7. The cannula device 1 is placed in an inserter, it is held in position inside the inserter by the friction between the insertion needle 3a which is unreleasably connected to the inserter and the cannula 3 of the cannula device. That the insertion needle 3 is unreleasably connected means that it can be molded into the plunger of the inserter. The cannula device of fig. 6 has two access openings, the insertion needle is placed through the first access opening covered by the membrane at position 4a and the second access opening is placed perpendicular to the first access opening and covered by the membrane at position 4b. When the cannula device is inserted into the base part it will be possible to access both openings as the receiving portion 7 has been provided with an opening corresponding to the second opening of the cannula device.

Fig. 7 shows a base part which can be used in combination with the cannula device of fig. 6 i.e. the base part provides several access openings for the delivery part. In fig. 7 one access is provided in a direction approximately perpendicular to the upper surface of the base part 9, according to the shown embodiment approximately would mean ±10° to perpendicular of the upper surface of the base part 9. The possible insertion angle is defined both by the position of the membrane but also by the angle of the walls of the inner cavity in the cannula device 1 therefore the connector needle 6 of the delivery part does not need to be exactly perpendicular to the upper surface of the base part 9. The second access opening is provided in a direction approximately parallel to the upper surface of the portion 9 of the base part i.e. the direction of the connection needle need not be exactly parallel to the upper surface of the base part but could deviate ±10° from parallel or as much as the walls of the inner cavity of the cannula device allow. In this embodiment the first connection angle, i.e. the connection through the first access opening, and the second connection angle, i.e. the connection through the second access opening, deviate from each other by around 90°, i.e. between 70° and 110°.

Fig. 8 shows a base part of similar type as the one shown in fig. 7 but in this embodiment the base part is of the form and size of a credit card without a separate mounting pad. The base part can be made by molding without a separate mounting pad and provided with an adhesive on the proximal side which secures the device to the user after mounting.

The openings of the attachment parts 11 are inclined towards the receiving portion in order for the delivery part to approach the receiving portion 7 in a correct angle. Actually the surfaces need not be inclined; they can have any form or direction directing the delivery part to connect with the base part or the cannula part 1 in a desired way. When joining the delivery part to the base part 9, the delivery part is first positioned on the attachments parts 11 in a position away from the receiving portion and then the delivery part slides along the attachment parts 11 until the final position is reached and a fluid connection is made between the delivery part and the cannula part 1. According to a similar embodiment the attachment parts 11 could comprise a single longish central part instead of two separate parts placed near the sides of the base part 9.

## Claims

1. A delivery device comprising:
- a cannula device (1) for mounting in a base part (9) comprising a housing (2) and at least one membrane (4) together defining at least one cavity (6), wherein the at least one cavity (6) comprises walls, the cannula device (1) further comprises a cannula (3) mounted in the housing (2) and in fluid communication with the at least one cavity (6), said cannula device (1) provided with means (5) for attaching the cannula device (1) to a base part (9);
- said base part (9) comprising a receiving portion (7) adapted to receive the cannula device (1), a portion (9) which can be placed on the skin of a patient and attachment parts (11); and
- a delivery part comprising parts corresponding to attachment parts (11) of the base part (9), at least a reservoir (13), and transporting means providing controlled transport of fluid from the at least a reservoir (13) to the patient, said parts corresponding to attachment parts (11) slidable along a track or surface from a first position to a second position, wherein a fluid connection between the delivery part and the cannula device is formed,
said delivery part connected to the base part (9) during use, wherein
a connection for transferring fluid from the at least a reservoir (13) of the delivery part to the base part (9) can be provided at different angles relative to the base part (9), said different angles defined by positioning of the at least one membrane (4) relative to the base part (9) or said walls of the at least one cavity of the cannula device (1) relative to the base part (9), wherein at least two connection angles deviate from each other by at least 40°, said cannula device (1) provided with guiding means (8) corresponding to guiding means (15) of an inserter device (14), and said guiding means (8) of the cannula device (1) secures the cannula device (1) when being moved towards the base part (9).

2. A delivery device according to claim 1, **characterized in that** the transporting means is a pump.

3. A delivery device according to claim 1 or 2 **characterized in that** at least two connection angles deviate from each other by at least 60°.

4. A delivery device according to any of claims 1-3, **characterized in that** the cannula device (1) is provided with more than one access opening to an inner cavity.

5. A delivery device according to any of claims 1-4 **characterized in that** the means (5) for attaching the cannula device (1) to the base part (9) is on the proximal side of the cannula device (1).

6. A delivery device according to claim 5 **characterized in that** the means (5) for attaching the cannula device (1) to the base part (9) comprises mechanical features cooperating with corresponding means (7a) on the base part (9).

7. A delivery device according to claim 5 or 6 **characterized in that** the means (5) for attaching the cannula device (1) to the base part (9) comprises parts (5) extending from the proximal surface of the cannula device (1) which parts (5) can pivot and thereby temporarily reduce the diameter formed by the edges of the parts (5) in at least one position.

8. A delivery device according to claim 5 or 6 **characterized in that** the means (5) for attaching the cannula device (1) to the base part (9) comprise an adhesive surface on the proximal surface of the cannula device (1) adhering to a corresponding surface of the base part (9).

9. A delivery device according to any preceding claim **characterized in that** the base part (9) comprises a body (1b) showing a smooth outer surface and having an inner cavity, the inner cavity is at the distal end covered with a wall (4) which can be penetrated by a needle (6) and at the proximal end of the inner cavity a cannula (3) is embedded, the outer proximal surface of the body (1b) is provided with means (5) for unreleasably attaching the cannula device (1) to a receiving portion (7).

10. A delivery device according to claim 5 **characterized in that** an unreleasable attachment between the receiving portion (7) and the cannula device (1) is formed automatically as the cannula device (1) is pushed against the receiving portion (7).

11. A delivery device according to claim 1, **characterized in that** the delivery part has more than one position relative to the attachment parts (11) and in a first position parts of the delivery part corresponding to the attachment parts (11) of the base part (9) is/are brought into contact with the attachments parts (11) and then the parts of the delivery part corresponding to the attachment parts (11) of the base part (9) slides along a track or a surface towards a second position where a fluid connection between the delivery part and the cannula device (1) is formed.

12. A system comprising: the delivery device according to any of claims 1-11; and an inserter device for insertion of the cannula device (1) in the base part (9) of the delivery device, said inserter device comprising a first insertion part (15) and a second insertion part (14), and an injection needle (3a) where
- the second insertion part (14) is connected to the injection needle (3a) and the injection needle (3a) is releasably combined with the cannula (3) of the cannula device (1),
- the first insertion part (15) covers the injection needle (3a) in a non-activated position,
- the first insertion part (15) engages with the second insertion part (14), and said first insertion part (15) is provided with guiding means interacting with corresponding guiding means of the second insertion part (14) for guiding a slidable movement of the first (15) and second (14) insertion parts in relation to each other, and
- the guiding means of the first (15) and the second (14) insertion part allows the injection needle (3a) to project beyond the first insertion part (15) when the insertion device is activated.

13. A system according to claim 12, where the cannula device (1) has a retracted position inside the first insertion part (15) and a forward position inside the first insertion part (15), and in the forward position the cannula (3) of the cannula device (1) extends beyond the open proximal end of the first insertion part (15).

14. A system according to claim 12 or 13, wherein the first insertion part (15) is provided with guiding means which means can be combined with means of a base part (9) being secured to the patient in order to create a well- defined insertion point and angle.

## Patentansprüche

1. Abgabevorrichtung, umfassend:
- eine Kanülenvorrichtung (1) zum Einbau in ein Basisteil (9), das ein Gehäuse (2) und wenigstens eine Membran (4) umfasst, die zusammen wenigstens einen Hohlraum (6) definieren, wobei der wenigstens eine Hohlraum (6) Wände umfasst, die Kanülenvorrichtung (1) ferner eine Kanüle (3) umfasst, die in dem Gehäuse (2) eingebaut ist und in Fluidverbindung mit dem wenigstens einen Hohlraum (6) steht, wobei die Kanülenvorrichtung (1) mit Mitteln (5) zum Befestigen der Kanülenvorrichtung (1) an einem Basisteil (9) versehen ist;
- wobei das Basisteil (9) einen Aufnahmeabschnitt (7) umfasst, der geeignet ist, die Kanülenvorrichtung (1) aufzunehmen, einen Abschnitt (9), der auf der Haut eines Patienten platziert werden kann, und Befestigungsteile (11); und
- ein Abgabeteil, das Teile umfasst, die mit Befestigungsteilen (11) des Basisteils (9) korrespondieren, wenigstens ein Reservoir (13), und Fördermittel, die einen kontrollierten Transport von Flüssigkeit aus dem wenigstens einen Reservoir (13) zu dem Patienten bereitstellen, wobei die Teile, die mit Befestigungsteilen (11) korrespondieren, entlang einer Spur oder Oberfläche von einer ersten Position zu einer zweiten Position verschiebbar sind, wobei eine Flüssigkeitsverbindung zwischen dem Abgabeteil und der Kanülenvorrichtung gebildet wird,
wobei das Abgabeteil während der Verwendung mit dem Basisteil (9) verbunden ist, wobei eine Verbindung zum Übertragen von Flüssigkeit von dem wenigstens einen Reservoir (13) des Abgabeteils zu dem Basisteil (9) in verschiedenen Winkeln relativ zu dem Basisteil (9) bereitgestellt werden kann, wobei die verschiedenen Winkel durch die Positionierung der wenigstens einen Membran (4) relativ zu dem Basisteil (9) oder der Wände des wenigstens einen Hohlraums der Kanülenvorrichtung (1) relativ zu dem Basisteil (9) definiert sind, wobei wenigstens zwei Verbindungswinkel um wenigstens 40° voneinander abweichen, wobei die Kanülenvorrichtung (1) mit Führungsmitteln (8) versehen ist, die mit Führungsmitteln (15) einer Einsetzvorrichtung (14) korrespondieren, und wobei die Führungsmittel (8) der Kanülenvorrichtung (1) die Kanülenvorrichtung (1) sichern, wenn sie zum Basisteil (9) hin bewegt wird.

2. Abgabevorrichtung nach Anspruch 1, **dadurch gekennzeichnet, dass** das Fördermittel eine Pumpe ist.

3. Abgabevorrichtung nach Anspruch 1 oder 2, **dadurch gekennzeichnet, dass** wenigstens zwei Anschlusswinkel um wenigstens 60° voneinander abweichen.

4. Abgabevorrichtung nach einem der Ansprüche 1 bis 3, **dadurch gekennzeichnet, dass** die Kanülenvorrichtung (1) mit mehr als einer Zugangsöffnung zu einem inneren Hohlraum versehen ist.

5. Abgabevorrichtung nach einem der Ansprüche 1 bis 4, **dadurch gekennzeichnet, dass** sich die Mittel (5) zum Befestigen der Kanülenvorrichtung (1) am Basisteil (9) auf der proximalen Seite der Kanülenvorrichtung (1) befinden.

6. Abgabevorrichtung nach Anspruch 5, **dadurch gekennzeichnet, dass** das Mittel (5) zum Befestigen der Kanülenvorrichtung (1) an dem Basisteil (9) mechanische Merkmale umfasst, die mit korrespondierenden Mitteln (7a) an dem Basisteil (9) zu s ammenwirken.

7. Abgabevorrichtung nach Anspruch 5 oder 6, **dadurch gekennzeichnet, dass** das Mittel (5) zur Befestigung der Kanülenvorrichtung (1) am Basisteil (9) Teile (5) umfasst, die sich von der proximalen Oberfläche der Kanülenvorrichtung (1) aus erstrecken, wobei die Teile (5) schwenkbar sind und dadurch den von den Kanten der Teile (5) gebildeten Durchmesser in wenigstens einer Position vorübergehend verringern können.

8. Abgabevorrichtung nach Anspruch 5 oder 6, **dadurch gekennzeichnet, dass** die Mittel (5) zur Befestigung der Kanülenvorrichtung (1) an dem Basisteil (9) eine Haftfläche auf der proximalen Oberfläche der Kanülenvorrichtung (1) umfassen, die an einer korrespondierenden Oberfläche des Basisteils (9) haftet.

9. Abgabevorrichtung nach einem der vorhergehenden Ansprüche, **dadurch gekennzeichnet, dass** das Basisteil (9) einen Körper (1b) umfasst, der eine glatte äußere Oberfläche aufweist und einen inneren Hohlraum aufweist, wobei der innere Hohlraum am distalen Ende mit einer Wand (4) abgedeckt ist, die von einer Nadel (6) durchdrungen werden kann, und am proximalen Ende des inneren Hohlraums eine Kanüle (3) eingebettet ist, wobei die äußere proximale Oberfläche des Körpers (1b) mit Mitteln (5) zum unlösbaren Befestigen der Kanülenvorrichtung (1) an einem Aufnahmeabschnitt (7) versehen ist.

10. Abgabevorrichtung nach Anspruch 5, **dadurch gekennzeichnet, dass** eine unlösbare Befestigung zwischen dem Aufnahmeabschnitt (7) und der Kanülenvorrichtung (1) automatisch gebildet wird, wenn die Kanülenvorrichtung (1) gegen den Aufnahmeabschnitt (7) gedrückt wird.

11. Abgabevorrichtung nach Anspruch 1, **dadurch gekennzeichnet, dass** das Abgabeteil mehr als eine Position relativ zu den Befestigungsteilen (11) hat und in einer ersten Position Teile des Abgabeteils, die mit den Befestigungsteilen (11) des Basisteils (9) korrespondieren, in Kontakt mit den Befestigungsteilen (11) gebracht wird/werden und dann die Teile des Abgabeteils, die mit den Befestigungsteilen (11) des Basisteils (9) korrespondieren, entlang einer Spur oder einer Oberfläche zu einer zweiten Position gleiten, wo eine Flüssigkeitsverbindung zwischen dem Abgabeteil und der Kanülenvorrichtung (1) gebildet wird.

12. System, umfassend: die Abgabevorrichtung nach einem der Ansprüche 1 bis 11; und eine Einsetzvorrichtung zum Einsetzen der Kanülenvorrichtung (1) in das Basisteil (9) der Abgabevorrichtung, wobei die Einsetzvorrichtung ein erstes Einlegeteil (15) und ein zweites Einlegeteil (14) sowie eine Injektionsnadel (3a) umfasst, wobei
- das zweite Einlegeteil (14) mit der Injektionsnadel (3a) verbunden ist und die Injektionsnadel (3a) lösbar mit der Kanüle (3) der Kanülenvorrichtung (1) verbunden ist,
- das erste Einlegeteil (15) die Injektionsnadel (3a) in einer nicht-aktivierten Position abdeckt,
- das erste Einlegeteil (15) in das zweite Einlegeteil (14) eingreift, und das erste Einlegeteil (15) mit einem Führungsmittel versehen ist, das mit einem korrespondierenden Führungsmittel des zweiten Einlegeteils (14) zusammenwirkt, um eine gleitende Bewegung des ersten (15) und des zweiten (14) Einlegeteils in Bezug zueinander zu führen, und
- das Führungsmittel des ersten (15) und des zweiten (14) Einlegeteils es der Injektionsnadel (3a) ermöglichen, über das erste Einlegeteil (15) hinauszuragen, wenn die Einlegevorrichtung aktiviert ist.

13. System nach Anspruch 12, wobei die Kanülenvorrichtung (1) eine eingefahrene Position innerhalb des ersten Einlegeteils (15) und eine vordere Position innerhalb des ersten Einlegeteils (15) aufweist und die Kanüle (3) der Kanülenvorrichtung (1) in der vorderen Position über das offene proximale Ende des ersten Einlegeteils (15) hinausragt.

14. System nach Anspruch 12 oder 13, wobei das erste Einlegeteil (15) mit Führungsmitteln versehen ist, die mit Mitteln eines am Patienten zu befestigenden Basisteils (9) kombiniert werden können, um einen wohldefinierten Eintrittspunkt und - winkel zu schaffen.

## Revendications

1. Dispositif de distribution comprenant :
- un dispositif (1) à canule à monter dans une pièce (9) de base comprenant un boîtier (2) et au moins une membrane (4) définissants ensemble au moins une cavité (6), dans lequel la au moins une cavité (6) comprend des parois, le dispositif (1) à canule comprenant en outre une canule (3) montée dans le boîtier (2) et en communication fluidique avec la au moins une cavité (6), le dispositif (1) à canule étant pourvu de moyens (5) de fixation du dispositif (1) à une pièce (9) de base ;
- la pièce (9) de base comprenant une partie (7) de réception conçue pour recevoir le dispositif (1) à canule, une partie (9) qui peut être placée sur la peau d'un patient et des pièces (11) de fixation ; et
- une pièce de distribution comprenant des pièces correspondant aux pièces (11) de fixation de la pièce (9) de base, au moins un réservoir (13) et un moyen de transport procurant un transport de fluide commandé du au moins un réservoir (13) au patient, les pièces correspondant à des pièces (11) de fixation pouvant glisser le long d'une piste ou une surface d'une première position à une deuxième position, une communication fluidique étant formée entre la pièce de distribution et le dispositif à canule,
la pièce de distribution étant reliée à la pièce (9) de base en utilisation, dans lequel une communication pour transférer du fluide du au moins un réservoir (13) de la pièce de distribution à la pièce (9) de base peut être prévue sous des angles différents par rapport à la pièce (9) de base, les angles différents étant définis en mettant la au moins une membrane (4) en position par rapport à la pièce (9) de base ou les parois de la au moins une cavité du dispositif (1) à canule par rapport à la pièce (9) de base, dans lequel au moins deux angles de liaison s'écartent l'un de l'autre d'au moins 40°, le dispositif (1) à canule étant pourvu de moyens (8) de guidage correspondants à des moyens (15) de guidage d'un dispositif (14) d'insertion et les moyens (8) de guidage du dispositif (1) à canule fixant le dispositif (1) à canule lorsqu'il est déplacé vers la pièce (9) de base.

2. Dispositif de distribution suivant la revendication 1, **caractérisé en ce que** le moyen de transport est une pompe.

3. Dispositif de distribution suivant la revendication 1 ou 2, **caractérisé en ce qu'**au moins deux angles de communication s'écartent l'un de l'autre d'au moins 60°.

4. Dispositif de distribution suivant l'une quelconque des revendications 1 à 3, **caractérisé en ce que** le dispositif (1) à canule est pourvu de plus d'une ouverture d'accès à une cavité intérieure.

5. Dispositif de distribution suivant l'une quelconque des revendications 1 à 4, **caractérisé en ce que** le moyen (5) de fixation du dispositif (1) à canule à la pièce (9) de base est du côté proximal du dispositif (1) à canule.

6. Dispositif de distribution suivant la revendication 5, **caractérisé en ce que** le moyen (5) de fixation du dispositif (1) à canule à la pièce (9) de base comprend des agencements mécaniques coopérants avec un moyen (7a) à correspondant sur la pièce (9) de base.

7. Dispositif de distribution suivant la revendication 5 ou 6, **caractérisé en ce que** le moyen (5) pour fixer le dispositif (1) à canule à la pièce (9) de base comprend des pièces (5) s'étendant à partir de la surface proximale du dispositif (1) à canule, lesquelles pièces (5) peuvent pivoter et réduire ainsi temporairement le diamètre formé par les bords des pièces (5) dans au moins une position.

8. Dispositif de distribution suivant la revendication 5 ou 6, **caractérisé en ce que** le moyen (5) de fixation du dispositif (1) à canule à la pièce (9) de base comprend une surface adhésive sur la surface proximale du dispositif (1) à canule adhérant à une surface correspondante de la pièce (9) de base.

9. Dispositif de distribution suivant n'importe quelle revendication précédente, **caractérisé en ce que** la pièce (9) de base comprend un corps (1b) ayant une surface extérieure lisse et ayant une cavité intérieure, la cavité intérieure est, à l'extrémité distale, recouverte d'une paroi (4) qui peut être pénétrée par une aiguille (6) et, à l'extrémité proximale de la cavité intérieure, est incorporée une canule (3), la surface proximale extérieure du corps (1b) étant pourvue d'un moyen (5) de fixation non détachable du dispositif (1) à canule à une partie (7) de réception.

10. Dispositif de distribution suivant la revendication 5, **caractérisé en ce que** la fixation non détachable entre la partie (7) de réception et le dispositif (1) à canule est formé automatiquement alors que le dispositif (1) est poussé sur la partie (7) de réception.

11. Dispositif de distribution suivant la revendication 1, **caractérisé en ce que** la pièce de distribution a plus qu'une position par rapport aux pièces (11) de fixation et, dans une première position des pièces de la pièce de distribution correspondants aux pièces (11) de fixation de la pièce (9) de base est/sont mises en contact avec les pièces (11) de fixation, puis les pièces de la pièce de distribution correspondants aux pièces (11) de fixation de la pièce (9) de base glissent sur une piste ou une surface vers une seconde position où une communication fluidique entre la pièce de distribution et le dispositif (1) à canule est formée.

12. Système comprenant : le dispositif de distribution suivant l'une quelconque des revendications 1 à 11 ; et un dispositif d'insertion pour insérer le dispositif (1) à canule dans la pièce (9) de base du dispositif de distribution, le dispositif d'insertion comprenant une première pièce (15) d'insertion et une deuxième pièce (14) d'insertion et une aiguille (3a) d'injection, dans lequel
- la deuxième pièce (14) d'insertion est reliée à l'aiguille (3a) d'injection et l'aiguille (3a) d'injection est combinée de manière détachable à la canule (3) du dispositif (1) à canule,
- la première pièce (15) d'insertion recouvre l'aiguille (3a) d'injection dans une position non activée,
- la première pièce (15) d'insertion s'enclenche avec la deuxième pièce (14) d'insertion et la première pièce (15) d'insertion est pourvue de moyens de guidage interagissant avec un moyen de guidage correspondant de la deuxième pièce (14) d'insertion pour guider un mouvement de coulissement de la première (15) et de la deuxième (14) pièce d'insertion l'une par rapport à l'autre, et
- le moyen de guidage de la première (15) et de la deuxième (14) pièce d'insertion permet à l'aiguille (3a) d'injection de faire saillie au-delà de la première pièce (15) d'insertion lorsque le dispositif d'insertion est activé.

13. Système suivant la revendication 12, dans lequel le dispositif (1) à canule a une position rétractée à l'intérieur de la première pièce (15) d'insertion et une position vers l'avant à l'intérieur de la première pièce (15) d'insertion et dans la position vers l'avant la canule (3) du dispositif (1) à canule s'étend au-delà de l'extrémité proximale ouverte de la première pièce (15) d'insertion.

14. Système suivant la revendication 12 ou 13, dans lequel la première pièce (15) d'insertion est pourvue d'un moyen de guidage, lequel moyen peut être combiné à un moyen d'une pièce (9) de base fixée au patient afin de créer un point et un angle d'insertion bien définis.
